# EUROPÄISCHE PATENTSCHRIFT

(11) **EP 0 893 138 B1**
(45) Veröffentlichungstag und Bekanntmachung des Hinweises auf die Patenterteilung: **26.05.2004**
(21) Anmeldenummer: 97110241.3
(22) Anmeldetag: 23.06.1997
(51) Int. Cl.: A61M 25/10

(54) **Katheteranordnung**
Catheter assembly
Ensemble cathéter

(43) Veröffentlichungstag der Anmeldung: 27.01.1999
(73) Patentinhaber: Schneider (Europe) GmbH, 8180 Bülach (CH)
(72) Erfinder: Schneider, Ernst, 8135 Langnau am Albis (CH)
(74) Vertreter: Degwert, Hartmut, Dipl.-Phys.

(56) Entgegenhaltungen:
- EP-A- 0 604 803
- WO-A-95/16487
- US-A- 5 320 604
- US-A- 5 415 636
- US-A- 5 462 529

## Beschreibung

Die Erfindung bezieht sich auf eine Katheteranordnung gemäß dem Oberbegriff des Patentanspruchs 1.

Eine solche Katheteranordnung ist aus der EP-A-0 080 436 bekannt. Bei dieser Katheteranordnung ist sowohl am distalen Ende des Außenkatheters als auch am distalen Ende des Innenkatheters ein Okklusionsballon angebracht, mit deren Hilfe eine Engstelle in einem Gefäß nach beiden Seiten hin abgedichtet werden kann. Durch die Verschiebbarkeit des Innenkatheters relativ zum Außenkatheter kann der Abstand der beiden Ballons verändert werden, so daß die Länge des abgedichteten Bereichs um die zu behandelnde Engstelle den jeweiligen Verhältnissen angepaßt werden kann. In dieser Katheteranordnung weist der Innenkatheter vier Lumina auf, von denen eines die Aufgabe des Inflationslumens für den auf dem Innenkatheter angebrachten Ballon übernimmt und zwei weitere Lumina Zuleitungs- bzw. Ableitungskanäle für die der Engstelle zuzuführende Behandlungsflüssigkeit sind. Das vierte, zentral im Innenkatheter angeordnete Lumen dient mit Hilfe eines Überlaufkanals als Perfusionslumen, das nach Art einer Bypass-Leitung während der Behandlung die abgedichtete Engstelle überbrückt und somit den Fluß der Körperflüssigkeit in dem Gefäß auch während der Behandlung aufrechterhält. Zwischen dem Außenkatheter und dem Innenkatheter ist ein Zwischenraum vorhanden, der als zusätzliches Lumen dient, über das der Engstellenbereich durch Zuführen und Ableiten einer Spülflüssigkeit behandelt werden kann. Der Innenkatheter dieser Anordnung hat einen komplizierten Aufbau und ist in der Herstellung somit teuer. Das zentrale Lumen ist distal nicht verschließbar und kann daher nicht als Infusionslumen dienen.

Eine weitere Katheteranordnung der oben geschilderten Art ist aus der US-A-4 655 746 bekannt. Bei dieser bekannten Katheteranordnung hat der Innenkatheter nur ein Lumen, das gleichzeitig als Führungsdrahtlumen und als Inflationslumen für den am distalen Katheterende angebrachten Okklusionsballon dient. Das Führungsdrahtlumen muß daher am distalen Katheterende permanent verschlossen sein, damit es gleichzeitig als Inflationslumen zum Einsatz kommen kann. Zum Zuführen der Behandlungsflüssigkeit zu dem von den beiden Ballonen abgedichteten Bereich steht nur der Zwischenraum zwischen dem Außenkatheter und dem Innenkatheter zur Verfügung. Optional ist es auch möglich, im Innenkatheter eine Zuleitung für die Behandlungsflüssigkeit vorzusehen, was allerdings zu einem komplizierteren Aufbau des Innenkatheters führt sowie mit einer Zunahme des Gesamtprofils der Katheteranordnung verbunden ist.

Bei einer aus der EP-B-0 309 469 bekannten Katheteranordnung werden zwei Okklusionsballons zur beiderseitigen Abdichtung einer zu behandelnden Engstelle vorgesehen, wobei die Zufuhr des Behandlungsmittels ausschließlich über den Zwischenraum zwischen dem Innenkatheter und dem Außenkatheter erfolgen kann. Ein gleichzeitiges Infundieren einer Behandlungsflüssigkeit und Absaugen aus dem Behandlungsbereich ist mit dieser Katheteranordnung nicht möglich.

Bei der ebenfalls mit zwei Okklusionsballons versehenen bekannten Katheteranordnung nach der US-A-5 279 546 sind im Außenkatheter insgesamt sechs Lumina vorhanden, wobei vier als Infusionslumen dienen können. Auch das Führungsdrahtlumen im Innenkatheter, der dreilumig ausgebildet ist, kann als Infusionslumen eingesetzt werden. Bei dieser kompliziert aufgebauten Katheteranordnung ist zwischen dem Außenkatheter und dem Innenkatheter kein Zwischenraum vorhanden, so daß hohe Reibungskräfte auftreten, die ein Verschieben des Innenkatheters relativ zum Außenkatheter schwierig machen. Zusätzliche Trennwände und Lumina für die elektrischen Anschlußleitungen eines Ultraschallgebers vergrößern den Gesamtquerschnitt dieser Katheteranordnung. Der Ultraschallgeber schränkt die Einstellbarkeit der Ballondistanz ein und kann das Einströmen des Behandlungsmittels in den Ballonzwischenraum behindern.

Aus US-A-5 462 529 ist eine Katheteranordnung zum Behandeln von Abschnitten Körperflüssigkeit führender Gefäße bekannt, die einen Innenkatheter aufweist, der mit einem Führungsdrahtlumen zur Aufnahme eines Führungsdrahtes und einem Inflationslumen für einen an seinem distalen Ende angebrachten Ballon versehen ist und am distalen Ende des Führungsdrahtlumens eine Öffnung aufweist. Außerdem umfaßt diese Katheteranordnung einen Außenkatheter, der mit einem Inflationslumen für einen an seinem distalen Ende angebrachten Ballon und mit einem Lumen versehen ist, in dem der Innenkatheter zur Veränderung des Abstandes der beiden Ballons verschiebbar angebracht ist und das einen größeren Querschnitt als der Innenkatheter aufweist, so daß zwischen der Außenwand des Innenkatheters und der Innenwand des Außenkatheters ein als Infusionslumen verwendbarer Querschnitt vorhanden ist, wobei der Außenkatheter in den zwischen den beiden Ballons liegenden Bereich mündet. Ferner steht das Führungsdrahtlumen des Innenkatheters über eine Öffnung in der Wand des Innenkatheters mit dem zwischen den beiden Ballons liegenden Bereich in Verbindung. Die Öffnung am distalen Ende des Führungsdrahtlumens ist so eng bemessen, daß zwar der Führungsdraht hindurchgleiten kann, aber keine Infusionsflüssigkeit hindurchtreten kann.

Aus WO 95/16487 (vgl. Figur 13a) ist ebenfalls eine Katheteranordnung der vorstehend beschriebenen Gattung bekannt, bei der einer der beiden Ballons als Dilatationsballon ausgebildet ist.

Der Erfindung liegt die Aufgabe zugrunde, eine Katheteranordnung der eingangs angegebenen Art zu schaffen, bei der Innen- und Außenkatheter leicht gegeneinander verschiebbar und die Ballondistanz über einen möglichst großen Längenbereich einstellbar sind. Außerdem soll die Katheteranordnung einen einfachen Aufbau aufweisen, der bei kleinem Gesamtprofil für die Zu- und Ableitung des Behandlungsmittels Lumina mit großen Querschnitten zur Verfügung stellt.

Diese Aufgabe wird gemäß der Erfindung mit den im kennzeichnenden Teil des Patentanspruchs 1 angegebenen Merkmalen gelöst. Bei der erfindungsgemäßen Katheteranordnung kann das Führungsdrahtlumen des Innenkatheters zusätzlich zu seiner Funktion als Führungsdrahtlumen auch als Infusionslumen zum Zuführen oder Abführen einer Behandlungsflüssigkeit ausgenutzt werden, da die Öffnungen in der Katheterwand im Bereich zwischen den beiden Ballons eine Strömungsverbindung zu dem zu behandelnden Gefäßabschnitt herstellen. Durch eine geeignete Wahl des Querschnitts des Führungsdrahtlumens wird diese Eignung dieses Lumens als Infusionslumen noch verstärkt. Damit das Führungsdrahtlumen auch tatsächlich als

Infusionslumen verwendet werden kann, ist an seinem distalen Ende ein Verschlußelement angebracht, das die Öffnung des Führungsdrahtlumens verschließen kann, damit die Behandlungsflüssigkeit nicht aus dieser Öffnung ausströmen kann, sondern durch die Öffnungen in den Bereich zwischen den beiden Ballons gelangt.

Die Ausbildung eines der Ballons als Dilatationsballon eröffnet neue Einsatzgebiete für die Katheteranordnung, wenn in dem zu behandelnden Gefäßabschnitt eine Engstelle vorliegt. Hierzu wird in einer Vorbehandlungsstufe der als Dilatationsballon ausgebildete Ballon verwendet, um die Engstelle mechanisch aufzuweiten. Wie dem Fachmann geläufig ist, muß der Ballon in seiner Funktion als Dilatationsballon anderen Kriterien entsprechen als ein reiner Okklusionsballon, der lediglich die Funktion hat, ein eine Körperflüssigkeit führendes Gefäß zu verschließen. Die besonderen Anforderungen an den Dilatationsballon betreffen dabei sowohl seine geometrischen Abmessungen als auch die Auswahl seines Materials.

Ein Ausführungsbeispiel der Erfindung wird nun unter Bezugnahme auf die Zeichnungen erläutert. In der Zeichnung zeigen:
- Figur 1 eine schematische Gesamtansicht der erfindungsgemäßen Katheteranordnung und
- Figur 2 einen Schnitt längs der Linie 2-2 von Figur 1.

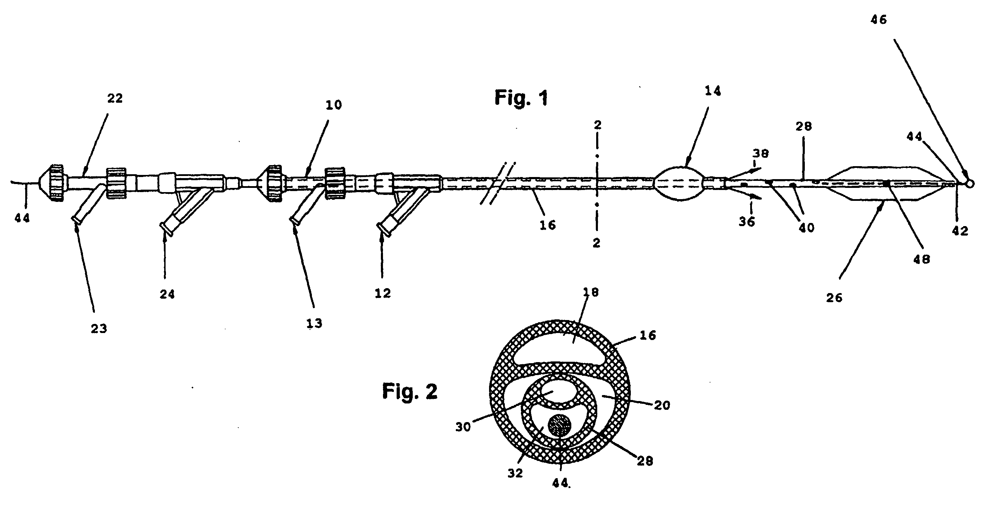

Die in Figur 1 dargestellte Katheteranordnung besteht aus einem Außenkatheter mit einem Y-Verbinder 10, einem Inflationsanschluß 12 zum Zuführen eines Druckmittels zu einem am distalen Ende dieses Außenkatheters angebrachten Okklusionsballon 14 sowie einem Katheterschaft 16. In der Schnittansicht von Figur 2 ist zu erkennen, daß der Schaft 16 des Außenkatheters zweilumig ausgeführt ist, wobei das Lumen 18 der Zuführung des Druckmittels zum Okklusionsballon 14 dient, während über das Lumen 20 einem zu behandelnden Gefäßabschnitt ein Behandlungsmittel, beispielsweise ein Thrombolythikum, zugeführt werden kann.

Die Katheteranordnung enthält ferner einen Innenkatheter mit einem Y-Verbinder 22, einem Inflationsanschluß 24 zum Zuführen eines Druckmittels zu einem am distalen Ende dieses Innenkatheters angebrachten Dilatationsballon 26 sowie einen Katheterschaft 28. Aus der Schnittansicht von Figur 2 geht hervor, daß auch der Schaft 28 des Innenkatheters zweilumig ausgeführt ist. Er enthält ein Lumen 30 zum Zuführen des Druckmittels zum Dilatationsballon 26 sowie ein Lumen 32, das der Aufnahme eines Führungsdrahtes 34 dient.

Das den Innenkatheter aufnehmende Lumen 20 des Außenkatheters weist einen wesentlich größeren Querschnitt als der Schaft 28 des Innenkatheters auf, so daß auch nach Einführen des Innenkatheters in das Lumen 20 ein großer Strömungsquerschnitt für die Zuführung der Behandlungsflüssigkeit zur Verfügung steht. Auch das den Führungsdraht 44 aufnehmende Lumen 32 des Innenkatheters ist im Querschnitt wesentlich größer als der Führungsdrahtquerschnitt, so daß auch dieses Lumen als Strömungsweg für die Behandlungsflüssigkeit verwendet werden kann. Dies gewährleistet auch die leichte Verschiebbarkeit von Innen- und Außenkatheter.

In Figur 1 zeigen die Pfeile 36, 38 an, wie die über einen Anschluß 13 am Y-Verbinder 10 zugeführte Behandlungsflüssigkeit aus dem Zwischenlumen in den Bereich zwischen den beiden Ballons gelangt. Damit auch das Führungsdrahtlumen zum Zuführen der Behandlungsflüssigkeit eingesetzt werden kann, sind im Bereich zwischen den beiden Ballons 14, 26 im Schaft des Innenkatheters Öffnungen 40 angebracht, durch die die Behandlungsflüssigkeit strömen kann. Damit die über einen Anschluß 23 am Y-Verbinder 22 zugeführte Behandlungsflüssigkeit, die durch das Führungsdrahtlumen 32 strömt, nicht an der Öffnung 42 am distalen Ende des Innenkatheters austritt, ist der Führungsdraht 44 an seinem Ende mit einem Verschlußelement 46 versehen, das sich durch Zurückziehen des Führungsdrahtes an die Öffnung 42 anlegen kann, so daß diese verschlossen wird.

Die beschriebene Katheteranordnung kann wie folgt zur Behandlung einer Engstelle eingesetzt werden. Zunächst wird die Katheteranordnung in dem eine Körperflüssigkeit führenden Gefäß so weit vorgeschoben, daß sich der Dilatationsballon 26 genau an der Engstelle befindet. Die Lage des Dilatationsballons 26 kann dabei wie üblich mit Hilfe eines innerhalb des Dilatationsballons 26 auf dem Schaft 28 angebrachten Metall-Markers 48 auf dem Röntgenschirm beobachtet werden. Durch Zuführen eines Druckmittels über den Anschluß 24 kann der Dilatationsballon 26 aufgeweitet werden, was zur Aufweitung der zu behandelnden Engstelle führt. Nachdem dieser Aufweitvorgang durchgeführt worden ist, wird das Druckmittel aus dem Dilatationsballon 26 zunächst abgelassen, und der Innenkatheter wird über die zu behandelnde Engstelle hinaus in das Gefäß geschoben, bis er sich hinter der Engstelle befindet. Der Okklusionsballon 14 bleibt dabei im Bereich vor der Engstelle. Nun wird den beiden Ballons über die Anschlüsse 24 und 12 ein Druckmittel zugeführt, so daß beide Ballons aufgeweitet werden und das Gefäß vor und hinter der Engstelle verschließen. Die Behandlungsflüssigkeit, die in der gewünschten Weise auf das die Engstelle bildende kalk- und fetthaltige Gewebe einwirkt, kann nun über den Anschluß 23 des Y-Verbinders 22 in den abgedichteten Bereich zwischen den beiden Ballons 14 und 26 eingeführt werden. Das Absaugen dieser Flüssigkeit kann über die Öffnungen 40 im Schaft 28 des Innenkatheters und über das Führungsdrahtlumen erfolgen. Natürlich ist es auch möglich, die Behandlungsflüssigkeit über das Führungsdrahtlumen 32 des Innenkatheters zuzuführen und über das Lumen 20 im Außenkatheter abzuführen.

Während der gesamten Behandlungsdauer muß natürlich der Führungsdraht 44 im Führungsdrahtlumen 32 so weit zurückgezogen werden, daß das Verschlußelement 46 in Anlage an die Öffnung 42 am distalen Ende des Innenkatheters kommt und diese Öffnung 42 verschließt. Nur dann kann das Führungsdrahtlumen 32 auch als Lumen zum Zuführen oder Abführen der Behandlungsflüssigkeit eingesetzt werden.

Nach Ablauf der Einwirkungsdauer der Behandlungsflüssigkeit auf das Engstellengewebe und nach Absaugen der Behandlungsflüssigkeit aus dem abgedichteten Bereich um die Engstelle wird das Druckmittel aus den beiden Ballons 14 und 26 abgelassen, so daß die Katheteranordnung wieder aus dem Gefäß entfernt werden kann.

Die beschriebene Katheteranordnung stellt aufgrund ihres geschilderten Aufbaus einen großen Strömungsquerschnitt für die Behandlungsflüssigkeit zur Verfügung, und sie hat dennoch einen geringen Gesamtquerschnitt, der ihr einfaches Platzieren auch in engen Gefäßen mit zu behandelnden Engstellen ermöglicht. Der Ballonabstand und damit die Länge des zu behandelnden Gefäßabschnitts sind über einen breiten Bereich einstellbar.

## Patentansprüche

1. Katheteranordnung zum Behandeln von Abschnitten Körperflüssigkeit führender Gefäße mit:
einem Innenkatheter, der mit einem Führungsdrahtlumen (32) zur Aufnahme eines Führungsdrahtes (44) und einem Inflationslumen (30) für einen an seinem distalen Ende angebrachten Ballon (26) versehen ist und am distalen Ende des Führungsdrahtlumens (32) eine Öffnung (42) aufweist,
einem Außenkatheter, der mit einem Inflationslumen (18) für einen an seinem distalen Ende angebrachten Ballon (14) und mit einem Lumen (20) versehen ist, in dem der Innenkatheter zur Veränderung des Abstandes der beiden Ballons (14, 26) verschiebbar angebracht ist und das einen größeren Querschnitt als der Innenkatheter aufweist, so daß zwischen der Außenwand des Innenkatheters und der Innenwand des Außenkatheters ein als Infusionslumen verwendbarer Querschnitt vorhanden ist, und in den zwischen den beiden Ballons (14, 26) liegenden Bereich mündet,
**dadurch gekennzeichnet,**
**daß** das Führungsdrahtlumen (32) des Innenkatheters über Öffnungen (40) in der Wand des Innenkatheters mit dem zwischen den beiden Ballons (14, 26) liegenden Bereich in Verbindung steht,
**daß** das Führungsdrahtlumen (32) im Querschnitt so dimensioniert ist, daß es auch bei liegendem Führungsdraht (44) als Infusionslumen verwendbar ist,
und **daß** der Führungsdraht (44) an seinem distalen Ende mit einem Verschlußelement (46) versehen ist, das die Öffnung (42) am distalen Ende des Führungsdrahtlumens (32) verschließen kann.

2. Katheteranordnung nach Anspruch 1, **dadurch gekennzeichnet, daß** einer der beiden Ballons (14, 26) als Dilatationsballon ausgebildet ist.

3. Katheteranordnung nach Anspruch 1 oder 2, **dadurch gekennzeichnet, daß** der am distalen Ende des Innenkatheters angebrachte Ballon (26) als Dilatationsballon ausgebildet ist.

## Claims

1. A catheter assembly for the treatment of sections of vessels carrying body fluid, comprising:
an inner catheter provided with a guide wire lumen (32) for receiving a guide wire (44) and an inflation lumen (30) for a balloon (26) attached to its distal end, and a port (42) located at the distal end of said guide wire lumen (32),
an outer catheter provided with an inflation lumen (18) for a balloon (14) attached to its distal end and a lumen (20) in which the inner catheter is applied shiftably for changing the spacing of said two balloons (14, 26) and which features a larger cross-section than that of said inner catheter so that between the outer wall of said inner catheter and the inner wall of said outer catheter a cross-section employable as an infusion lumen exists, and ports in the portion sited between said two balloons (14, 26),
**characterized in that**
said guide wire lumen (32) of said inner catheter is connected to said portion sited between said two balloons (14, 26) via ports (40) in said inner catheter wall,
said guide wire lumen (32) is dimensioned cross-sectionally such that it can be used as an infusion lumen also when said guide wire (44) is in the lying position,
and said guide wire (44) is provided at its distal end with an occlusion element (46) which can close said port (42) located at the distal end of said guide wire lumen (32).

2. The catheter assembly according to claim 1, **characterized in that** one of said two balloons (14, 26) is configured as a dilatation balloon.

3. The catheter assembly according to claim 1 or 2, **characterized in that** said balloon (26) attached to the distal end of said inner catheter is configured as a dilatation balloon.

## Revendications

1. Agencement de cathéter pour le traitement de portions de vaisseaux transportant du liquide corporel, comportant
un cathéter intérieur qui est pourvu d'une lumière de fil de guidage (32) pour recevoir un fil de guidage (44) et d'une lumière d'inflation (30) pour un ballon (26) monté à son extrémité distale, et qui présente une ouverture (42) à l'extrémité distale de la lumière de fil de guidage (32),
un cathéter extérieur qui est pourvu d'une lumière d'inflation (18) pour un ballon (14) monté à son extrémité distale et d'une lumière (20) dans laquelle le cathéter intérieur est monté à coulissement pour modifier la distance entre les deux ballons (14, 26) et qui présente une section transversale supérieure à celle du cathéter intérieur, de telle sorte qu'il y a entre la paroi extérieure du cathéter intérieur et la paroi intérieure du cathéter extérieur une section transversale pouvant être utilisée à titre de lumière de perfusion, et qui débouche dans la zone située entre les deux ballons (14, 26),
**caractérisé en ce que**
la lumière de fil de guidage (32) du cathéter intérieur communique avec la zone située entre les deux ballons (14, 26) via des orifices (40) dans la paroi du cathéter intérieur,
**en ce que** la lumière de fil de guidage (32) a une section transversale de dimensions telles qu'elle peut être utilisée aussi à titre de lumière de perfusion lorsque le fil de guidage (44) est en position couchée,
et **en ce que** le fil de guidage (44) est pourvu, à son extrémité distale, d'un élément de fermeture (46) qui peut fermer l'orifice (42) à l'extrémité distale de la lumière de fil de guidage (32).

2. Agencement de cathéter selon la revendication 1, **caractérisé en ce qu'**un des deux ballons (14, 26) est réalisé sous forme de ballon de dilatation.

3. Agencement de cathéter selon la revendication 1 ou 2, **caractérisé en ce que** le ballon (26) monté à l'extrémité distale du cathéter intérieur est réalisé sous forme de ballon de dilatation.
